# EUROPÄISCHE PATENTSCHRIFT

(11) **EP 3 697 282 B1**
(45) Veröffentlichungstag und Bekanntmachung des Hinweises auf die Patenterteilung: **18.05.2022**
(21) Anmeldenummer: 18782993.2
(22) Anmeldetag: 05.10.2018
(51) Int. Cl.: A61B 1/00, G02B 23/24, G02B 5/04, G02B 17/04, A61B 1/04

(54) **UMLENKPRISMENBAUGRUPPE FÜR EIN ENDOSKOP UND ENDOSKOP MIT SEITLICHER BLICKRICHTUNG**
DEFLECTION PRISM ASSEMBLY FOR AN ENDOSCOPE AND ENDOSCOPE HAVING A LATERAL VIEWING DIRECTION
MODULE DE PRISME DÉVIATEUR DESTINÉ À UN ENDOSCOPE ET ENDOSCOPE POURVU D'UN SENS DE VISION LATÉRALE

(30) Priorität: 20.10.2017 DE 102017124593
(43) Veröffentlichungstag der Anmeldung: 26.08.2020
(73) Patentinhaber: OLYMPUS Winter & Ibe GmbH, 22045 Hamburg (DE)
(72) Erfinder: WIETERS, Martin, 22885 Barsbüttel (DE); THÜMEN, Alrun, 22043 Hamburg (DE)
(74) Vertreter: Seemann & Partner Patentanwälte mbB
(86) Internationale Anmeldenummer: PCT/EP2018/077182
(87) Internationale Veröffentlichungsnummer: WO 2019/076654

(56) Entgegenhaltungen:
- EP-A1- 2 730 210
- CN-U- 204 009 189
- DE-A1- 2 458 306
- DE-A1-102011 078 968
- DE-A1-102011 090 132
- DE-A1-102014 202 612
- DE-A1-102014 202 669
- DE-A1-102015 101 624
- JP-A- 2014 119 474
- US-B1- 7 280 283

## Beschreibung

Die Erfindung betrifft eine Umlenkprismenbaugruppe für ein Endoskop und ein Endoskop mit seitlicher Blickrichtung.

In der Endoskopie ist es häufig sinnvoll, Endoskope einzusetzen, deren Blickrichtungen von 0°, also einem Geradeausblick, abweichen. Man spricht in diesem Fall von Endoskopen mit seitlicher Blickrichtung.

Ein solches Endoskop mit seitlicher Blickrichtung ist beispielsweise in DE 10 2011 090 132 A1 offenbart.

In Endoskopen, insbesondere Videoendoskopen, dieser Art kommen für gewöhnlich distale Umlenkprismen zum Einsatz, die aus mehreren Teilprismen aufgebaut sind. Die Aufgabe eines Umlenkprismas besteht darin, das schräg einfallende Licht so umzulenken, dass es nach dem Austritt aus dem Umlenkprisma parallel zur Endoskopachse verläuft.

Zur Fixierung des Umlenkprismas im Endoskop ist dieses in einem Prismenhalter aufgenommen. Dieser Prismenhalter ist für gewöhnlich zylinderförmig und umschließt das Umlenkprisma vollständig. Umlenkprisma und Prismenhalter bilden eine Umlenkprismenbaugruppe.

Um verbesserte optische Eigenschaften und ein größeres Blickfeld zu erreichen, kann ein größeres Umlenkprisma eingesetzt werden, da dadurch der Bereich, aus dem Licht in das Endoskop einfallen kann, vergrößert wird.

Vor allem bei medizinischen Endoskopen ist es aber von großer Bedeutung, dass der Außendurchmesser des Endoskopschafts möglichst klein ist. Dies ermöglicht minimalinvasive endoskopische Untersuchungen von Patienten.

Ein kleiner Außendurchmesser des Endoskopschafts erfordert aber, dass auch die Umlenkprismenbaugruppe möglichst klein ist. Somit stehen die Anforderungen an das Endoskop, wonach dieses einerseits eine hohe Bildqualität und eine große Lichtstärke und andererseits einen möglichst kleinen Außendurchmesser aufweisen soll, im Konflikt zueinander.

Aus DE 10 2015 010 624 A1 ist ein Endoskop mit seitlicher Blickrichtung bekannt. Es umfasst einen Schaft mit einem Hüllrohr und einem darin angeordneten Faserrohr, wobei ein Objektiv mit einer Bildaufnahmeeinheit im Schaft angeordnet ist. Eine Prismenbaugruppe dient zur Umlenkung des Strahlengangs. Die Prismenbaugruppe ist exzentrisch zu dem Objektiv angeordnet.

Aus EP 2 730 210 A1 ist ein Endoskop mit seitlicher Blickrichtung bekannt. Das Endoskop umfasst einen Hohlkörper, in dessen Endbereich ein Bildaufnahmesystem angeordnet ist. Bildeintrittsseitig weist dieses ein optisches Linsensystem, einen zylinderförmigen Abschnitt und anschließend einen Bildsensor auf, der das von dem optischen Linsensystem kommende Bild umwandelt. Die optische Achse des Linsensystems erstreckt sich in Richtung der Längsachse des Hohlkörpers, wobei der Außendurchmesser des zylinderförmigen Abschnitts des Linsensystems geringer ist als der Innendurchmesser des Hohlkörpers. Zwischen einer Innenseite des Hohlkörpers und der Außenseite des optischen Linsensystems ist ein Zwischenraum vorhanden.

Die Aufgabe der Erfindung besteht folglich darin, eine Umlenkprismenbaugruppe für ein Endoskop sowie ein Endoskop bereitzustellen, mit der/dem eine Verbesserung der optischen Eigenschaften erreicht wird, ohne den Außendurchmesser des Endoskopschafts zu vergrößern.

Diese Aufgabe wird gelöst durch eine Umlenkprismenbaugruppe für ein Endoskop mit seitlicher Blickrichtung, umfassend einen Prismenhalter und ein Umlenkprisma, welches in dem Prismenhalter aufgenommen ist, wobei das Umlenkprisma eine Lichtaustrittsfläche und eine zu dieser schräg angeordnete, gegenüberliegende Lichteintrittsfläche aufweist, zwischen denen sich eine Mantelfläche erstreckt, die dadurch weitergebildet ist, dass der Prismenhalter das Umlenkprisma dergestalt aufnimmt, dass der Prismenhalter die Mantelfläche des Umlenkprismas bereichsweise umgibt, wobei der Prismenhalter einen ersten Teil und einen zweiten Teil umfasst, wobei sich der erste Teil entlang einer gesamten Länge des Umlenkprismas erstreckt und sich der zweite Teil entlang eines Abschnitts der Länge des Umlenkprismas erstreckt, wobei die Länge eine Ausdehnung des Umlenkprismas in Richtung einer Normalen auf der Lichtaustrittsebene ist, wobei die Lichteintrittsfläche durch die schräge Anordnung eine bezüglich der Lichtaustrittsfläche nahe Teilfläche und eine entfernte Teilfläche aufweist, wobei sich der erste Teil des Prismenhalters von der Lichtaustrittsfläche zur nahen Teilfläche erstreckt und sich der zweite Teil des Prismenhalters von der Lichtaustrittsfläche in Richtung der entfernten Teilfläche erstreckt, wobei die Umlenkprismenbaugruppe dadurch fortgebildet ist, dass der zweite Teil des Prismenhalters eine obere Haltefläche umfasst, wobei eine Reflexionsfläche des Umlenkprismas an der oberen Haltefläche fixiert ist.

Die Lichteintrittsfläche ist so ausgerichtet, dass der Winkel zwischen Lichteintrittsfläche und Lichtaustrittsfläche der schrägen Blickrichtung des Endoskops entspricht. Die Mantelfläche erstreckt sich zwischen Lichteintrittsfläche und Lichtaustrittsfläche. Alle Bereiche der Oberfläche des Umlenkprismas, die nicht zur Lichteintrittsfläche oder Lichtaustrittsfläche gehören, sind somit Teil der Mantelfläche. Unter einem bereichsweisen Umschließen wird im Kontext der vorliegenden Beschreibung verstanden, dass im Vergleich zu einem zylinderförmigen Prismenhalter Bereiche des Prismenhalters ausgespart sind.

Vorteilhaft wird das Umlenkprisma in Bereichen vergrößert, die nicht vom Prismenhalter umgeben sind. Das Umlenkprisma wird dabei um genau das Volumen vergrößert, welches durch das nur bereichsweise Umschließen des Umlenkprismas ausgespart wurde. Durch diese Vergrößerung des Umlenkprismas kommt es zu keiner Zunahme des Umfangs der Umlenkprismenbaugruppe und damit zu keiner Zunahme des Durchmessers des Endoskopschafts. Gleichzeitig fällt durch die Vergrößerung des Umlenkprismas mehr Licht in das Endoskop ein, so dass sich die optischen Eigenschaften des Endoskops verbessern und sich die Lichtstärke des Endoskops vergrößert.

Vorteilhaft umschließt der Prismenhalter die Mantelfläche nur in den Bereichen, die für eine sichere Fixierung und eine genaue Ausrichtung des Umlenkprismas in dem Prismenhalter notwendig sind.

Vorzugsweise ist der Prismenhalter derart ausgestaltet, dass dieser das Umlenkprisma an keinem der möglichen Außenumfänge des Umlenkprismas vollständig umschließt, wobei die Außenumfänge auf der Mantelfläche des Umlenkprismas parallel zur Lichtaustrittsfläche verlaufen.

Für eine genaue Ausrichtung des Umlenkprismas in dem Prismenhalter ist es nicht erforderlich, dass der Prismenhalter die Mantelfläche entlang eines vollständigen Außenumfangs umschließt. Insbesondere können somit Bereiche des Prismenhalters vollständig ausgespart werden, die sich entlang der Mantelfläche von Lichteintrittsfläche zur Lichtaustrittsfläche erstrecken. Vorteilhaft wird dadurch eine platzsparende Bauweise des Prismenhalters realisiert. Durch Vergrößerung des Umlenkprismas in den Bereichen, die nicht vom Prismenhalter umschlossen sind, werden die optischen Eigenschaften des Endoskops verbessert.

Der Prismenhalter umfasst einen ersten Teil und einen zweiten Teil, wobei sich der erste Teil entlang einer gesamten Länge des Umlenkprismas erstreckt und sich der zweite Teil entlang eines Abschnitts der Länge des Umlenkprismas erstreckt, wobei die Länge eine Ausdehnung des Umlenkprismas in Richtung einer Normalen auf der Lichtaustrittsebene ist.

Der erste Teil des Prismenhalters erstreckt sich somit entlang der gesamten Länge der Mantelfläche zwischen Lichtaustrittsfläche und Lichteintrittsfläche. Dadurch eignet sich der erste Teil des Prismenhalters für die Fixierung des Umlenkprismas am Prismenhalter. Vorteilhaft umschließt der erste Teil die Mantelfläche des Umlenkprismas nur in dem Winkelbereich des Außenumfangs, der für eine sichere Fixierung notwendig ist. Beispielsweise umschließt der erste Teil des Prismenhalters die Mantelfläche nur auf etwa 150° entlang des Außenumfangs.

Der zweite Teil erstreckt sich nur entlang eines Abschnitts der Länge der Mantelfläche, wobei sich dieser Abschnitt von der Lichtaustrittsfläche in Richtung der Lichteintrittsfläche erstreckt. Zwischen Lichteintrittsfläche und dem zweiten Teil des Prismenhalters ist die Mantelfläche somit nicht von dem Prismenhalter umgeben. In diesem Bereich kann das Umlenkprisma vergrößert werden, um die optischen Eigenschaften des Endoskops weiter zu verbessern.

Die Lichteintrittsfläche weist durch die schräge Anordnung eine bezüglich der Lichtaustrittsfläche nahe Teilfläche und eine entfernte Teilfläche auf, wobei sich der erste Teil des Prismenhalters von der Lichtaustrittsfläche zur nahen Teilfläche erstreckt und sich der zweite Teil des Prismenhalters von der Lichtaustrittsfläche in Richtung der entfernten Teilfläche erstreckt.

Die schräge Anordnung der Lichteintrittsfläche hat zur Folge, dass die Reflexionen des einfallenden Lichts im Umlenkprisma im Wesentlichen im oberen Teil des Umlenkprismas stattfinden. Der obere Teil des Umlenkprismas ist dabei der Teil des Umlenkprismas, der sich von der entfernten Teilfläche zur Reflexionsfläche erstreckt, wobei die Reflexionsfläche der Teil der Mantelfläche ist, an der einfallendes Licht ein erstes Mal reflektiert wird. Der untere Teil des Umlenkprismas erstreckt sich hingegen von der nahen Teilfläche zur Lichtaustrittsfläche.

Der erste Teil des Prismenhalters umgibt somit bereichsweise die Mantelfläche im unteren Teil des Umlenkprismas, während der zweite Teil des Prismenhalters bereichsweise die Mantelfläche im oberen Teil des Umlenkprismas umgibt.

Da der Strahlengang des einfallenden Lichts im Wesentlichen im oberen Teil des Umlenkprismas verläuft, kann der untere Teil zur Fixierung verwendet werden. Der erste Teil des Prismenhalters, der diesen unteren Teil des Umlenkprismas umgibt, erstreckt sich daher entlang der gesamten Länge der Mantelfläche, so dass eine sichere Fixierung des Umlenkprismas an der Mantelfläche ermöglicht wird.

Um eine große Lichtstärke des Endoskops zu erreichen, wird der Prismenhalter im oberen Teil des Umlenkprismas bereichsweise ausgespart und das Umlenkprisma in diesem Bereich vergrößert. Aus diesem Grund erstreckt sich der zweite Teil des Prismenhalters nur entlang eines Abschnitts der Länge der Mantelfläche.

Erfindungsgemäß umfasst der zweite Teil des Prismenhalters eine obere Haltefläche, wobei eine Reflexionsfläche des Umlenkprismas an der oberen Haltefläche fixiert ist.

Da das einfallende Licht an der Reflexionsfläche reflektiert wird, wird der Bereich hinter der Reflexionsfläche nicht optisch benötigt. In diesem Bereich ist der zweite Teil des Prismenhalters angeordnet.

Durch eine solche Anordnung des zweiten Teils des Prismenhalters kann dieser zur Fixierung des Umlenkprismas verwendet werden, ohne dass eine negative Beeinflussung der optischen Eigenschaften des Endoskops auftritt. Dazu ist eine Fläche des zweiten Teils als obere Haltefläche ausgebildet, an der beispielsweise mittels Klebstoff die Reflexionsfläche fixiert wird.

Bevorzugt umfasst der erste Teil des Prismenhalters eine untere Haltefläche und das Umlenkprisma eine formkomplementäre Bodenfläche, wobei die untere Haltefläche und die Bodenfläche senkrecht zur Lichtaustrittsfläche angeordnet sind und die Bodenfläche an der unteren Haltefläche fixiert ist.

Da der untere Teil des Umlenkprismas optisch nicht genutzt wird, wird dieser zur Fixierung des Umlenkprismas an dem Prismenhalter verwendet. Dazu wird das Umlenkprisma, beispielsweise durch Abschleifen, so gestaltet, dass es eine zur Austrittsfläche senkrechte Bodenfläche aufweist. Der Prismenhalter weist eine formkomplementäre Haltefläche auf, an der die Bodenfläche fixiert wird. Diese Fixierung wird beispielsweise mittels eines Klebstoffs realisiert.

Vorteilhaft ist das Umlenkprisma sowohl an der oberen Haltefläche des zweiten Teils des Prismenhalters als auch an der unteren Haltefläche des ersten Teils des Prismenhalters fixiert. Auf diese Weise wird eine sichere Fixierung des Umlenkprismas im Prismenhalter erreicht.

Vorzugsweise umfasst der erste Teil des Prismenhalters wenigstens zwei aneinander angrenzende untere Halteflächen und das Umlenkprisma den unteren Halteflächen zugeordnete Bodenflächen.

Weist das Umlenkprisma nur eine einzelne Bodenfläche auf, so ist die Fixierung des Umlenkprismas an dem Prismenhalter anfällig gegenüber Scherkräften, die seitlich auf das Umlenkprisma wirken. Dadurch kann es unter Umständen zu einem Verrutschen des Umlenkprismas im Prismenhalter kommen, was eine Verschlechterung der optischen Eigenschaften des Endoskops zur Folge hätte. Um diesen Scherkräften entgegenzuwirken, ist die Verwendung mehrerer Bodenflächen und mehrerer unterer Halteflächen sinnvoll. Insbesondere schließen diese Bodenflächen (und somit auch die formkomplementären unteren Halteflächen) einen Winkel ein. Gleichzeitig sind alle Bodenflächen senkrecht zu der Lichtaustrittsfläche orientiert.

Vorzugsweise liegt ein Summenwinkel, also die Summe der einzelnen Winkel der aneinander angrenzenden unteren Halteflächen, zwischen 60° und 120°, insbesondere bei etwa 90°.

Zur Berechnung des Summenwinkels wird bei allen aneinander angrenzenden unteren Halteflächen der Winkel zwischen den aneinander angrenzenden Flächen bestimmt und diese Winkel aufsummiert.

Um den Scherkräften entgegenzuwirken, wären zwei untere Halteflächen, die im Winkel von 90° aufeinander stehen, möglich. Allerdings bedeutet die Verwendung von zwei Flächen, dass der erste Teil des Prismenhalters so groß gestaltet werden muss, dass der erste Teil des Prismenhalters den Außenumfang der Mantelfläche in einem Winkelbereich von etwa 180° umgibt.

Dies könnte negative Auswirkungen auf die optischen Eigenschaften des Endoskops haben und erschwert außerdem eine Zentrierung des Umlenkprismas, da hierfür ein möglichst großer Außenumfang der Mantelfläche benötigt wird, der nicht vom Prismenhalter umgeben ist.

Es muss daher ein Kompromiss zwischen einem möglichst großen Außenumfang der Mantelfläche des Umlenkprismas und einem möglichst großen Widerstand gegenüber Scherkräften gefunden werden. Ein solcher Kompromiss ist beispielsweise die Verwendung von drei unteren Halteflächen, die jeweils in einem Winkel von zumindest näherungsweise 45° zueinander stehen. Dies ermöglicht, einen Summenwinkel von etwa 90° zu erhalten und gleichzeitig einen Außenumfang der Mantelfläche, welcher nicht von dem ersten Teil des Prismenhalters umgeben ist, von etwa 210° zu realisieren.

Vorzugsweise sind der erste Teil und der zweite Teil durch einen Anschlag des Prismenhalters verbunden, wobei der Anschlag insbesondere ringförmig ist. Besonders vorzugsweise umfasst der Anschlag eine Anlagefläche, die zur Lichtaustrittsfläche des Umlenkprismas planparallel ist, wobei die Lichtaustrittsfläche bereichsweise an der Anlagefläche anliegt.

Der Anschlag ist proximal des Umlenkprismas angeordnet. Obwohl er eine ringförmige Form hat, umschließt er das Umlenkprisma daher nicht.

Durch das Anlegen der Lichtaustrittsfläche an der Anlagefläche wird eine Verkippung des Umlenkprismas minimiert. Die Anlagefläche ist, genau wie die Lichtaustrittsfläche, senkrecht zur Endoskopachse angeordnet. Dies ermöglicht eine genaue Ausrichtung der Neigung des Umlenkprismas bezüglich der Endoskopachse. Vorteilhafterweise liegt die Anlagefläche nur an dem Bereich der Lichtaustrittsfläche an, der optisch nicht genutzt wird.

Vorzugsweise ist zwischen unterer Haltefläche und zugeordneter Bodenfläche und/oder zwischen oberer Haltefläche und zugeordneter Reflexionsfläche ein Klebespalt mit einer Spaltbreite ausgebildet, wobei die Spaltbreite so groß ist, dass unter Berücksichtigung von Fertigungstoleranzen die untere Haltefläche an keiner Stelle an der Bodenfläche und/oder die obere Haltefläche an keiner Stelle an der Reflexionsfläche anliegt, wenn die Lichtaustrittsfläche bereichsweise an der Anlagefläche anliegt.

Da die Ausrichtung der Neigung über das Anlegen der Lichtaustrittsfläche an die Anlagefläche erfolgt, ist es vorteilhaft, wenn das Umlenkprisma an keiner anderen Stelle an dem Prismenhalter anliegt, da dies sich negativ auf die Ausrichtung auswirken würde.

Folglich werden das Umlenkprisma und der Prismenhalter so gefertigt, dass zwischen oberer Haltefläche und zugeordneter Reflexionsfläche und zwischen den unteren Halteflächen und den zugeordneten Bodenflächen jeweils ein Spalt besteht, so dass sich Umlenkprisma und Prismenhalter nur im Bereich der Anlagefläche berühren. Die Spalten werden insbesondere als Klebespalten zur Fixierung genutzt.

Vorzugsweise ist wenigstens ein Drittel, insbesondere wenigstens zwei Drittel, eines Außenumfangs der Mantelfläche kreisförmig. Weiterhin vorzugsweise ist wenigstens ein Drittel eines Außenumfangs des Prismenhalters, insbesondere ein vollständiger Außenumfang des Prismenhalters, kreisförmig.

Vorteilhaft haben der Außenumfang der Mantelfläche und des Prismenhalters den gleichen Radius.

Durch den kreisförmigen Außenumfang der Mantelfläche und des Prismenhalters können diese in Bezug auf die Endoskopachse beim Einbau in ein Endoskop ausgerichtet werden. Dabei ermöglicht der zumindest teilweise kreisförmige Außenumfang, dass der Außenumfang des Umlenkprismas und des Prismenhalters miteinander in Deckung gebracht und anschließend bezüglich der Endoskopachse ausgerichtet, d.h. zentriert, werden. Eine solche Ausrichtung des Umlenkprismas und des Prismenhalters verhindert Vignettierung und Bildbeschnitt und sorgt somit für eine Verbesserung der Bildqualität.

Die Aufgabe wird außerdem gelöst durch ein Endoskop, umfassend eine Umlenkprismenbaugruppe nach einer der zuvor genannten Ausführungsformen.

Auf das Endoskop treffen gleiche oder ähnliche Vorteile zu, wie sie bereits bezüglich der Umlenkprismenbaugruppe erläutert wurden, so dass auf Wiederholungen verzichtet werden soll.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematisch vereinfachte, perspektivische Darstellung eines Endoskops,
- Fig. 2: einen schematisch vereinfachten Längsschnitt durch eine Umlenkprismenbaugruppe mit einer Eintrittslinse und einer Austrittslinse nach dem Stand der Technik,
- Fig. 3: einen schematisch vereinfachten Längsschnitt durch eine erfindungsgemäße Umlenkprismenbaugruppe mit einer Eintrittslinse und einer Austrittslinse,
- Fig. 4a: einen schematisch vereinfachten Querschnitt durch eine erfindungsgemäße Umlenkprismenbaugruppe im Bereich der Lichteintrittsfläche,
- Fig. 4b: einen schematisch vereinfachten Querschnitt durch eine erfindungsgemäße Umlenkprismenbaugruppe im Bereich der Lichtaustrittslinse,
- Fig. 5: eine schematisch vereinfachte, perspektivische Darstellung einer erfindungsgemäßen Umlenkprismenbaugruppe.

In den Zeichnungen sind jeweils gleiche oder gleichartige Elemente und/oder Teile mit denselben Bezugsziffern versehen, so dass von einer erneuten Vorstellung jeweils abgesehen wird.

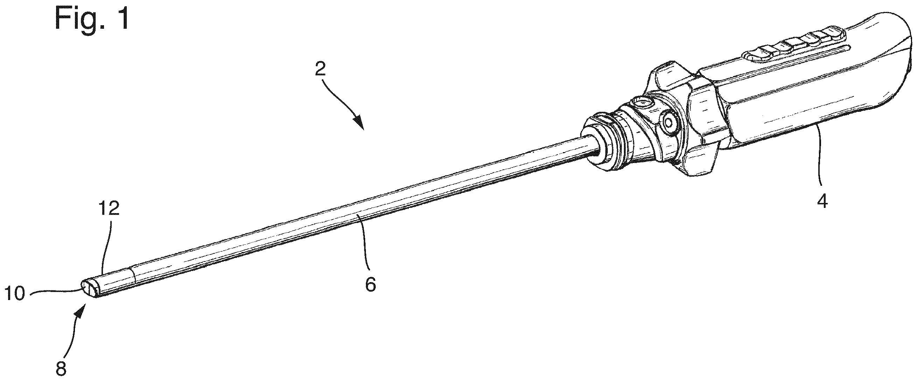

Fig. 1 zeigt ein Endoskop 2 mit seitlicher Blickrichtung. An einem proximalen Ende des Endoskops 2 befindet sich ein Handgriff 4, an welchen sich ein Schaft 6 anschließt. An einem distalen Ende 8 des Schafts 6 befindet sich ein Eintrittsfenster 10, durch welches Lichtbündel aus einem distal vor dem distalen Ende 8 gelegenen Beobachtungs- oder Operationsfeld in das Innere des Schafts 6 eintreten. In einem distalen Endbereich 12 des Schafts 6 ist ein Umlenkprisma als Teil einer Umlenkprismenbaugruppe innerhalb des Schafts 6 angeordnet.

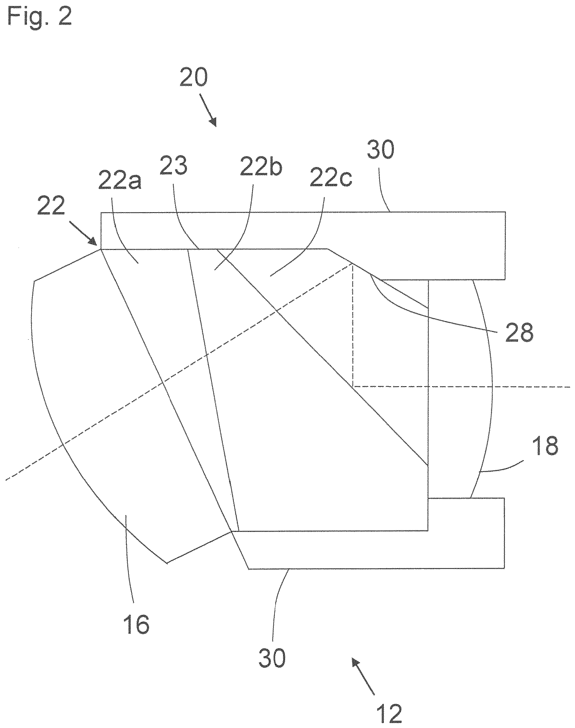

Fig. 2 zeigt schematisch eine Umlenkprismenbaugruppe 20 nach dem Stand der Technik. Die Umlenkprismenbaugruppe 20 umfasst ein Umlenkprisma 22, welches aus drei Teilprismen 22a, 22b, 22c besteht, und einen zylinderförmigen Prismenhalter 30. Der Prismenhalter 30 umschließt eine Mantelfläche 23 des Umlenkprismas 22 vollständig. Es sind ebenfalls eine Eintrittslinse 16 und eine Austrittslinse 18 gezeigt, welche jedoch nicht Bestandteil der Umlenkprismenbaugruppe 20 sind.

Aus einem Beobachtungsraum einfallendes Licht, welches durch eine gestrichelte Linie dargestellt ist, tritt durch das in Fig. 2 nicht dargestellte Eintrittsfenster 10 und die Eintrittslinse 16 in das Umlenkprisma 22 ein. An der Reflexionsfläche 28 wird das Licht ein erstes Mal reflektiert, bevor es ein zweites Mal an der Grenzfläche zwischen dem zweiten Teilprisma 22b und dem dritten Teilprisma 22c im Wesentlichen in eine Richtung parallel zur Endoskopachse reflektiert wird. Das Licht wird weiter über die Austrittslinse 18 in Richtung des Endoskopinneren geleitet.

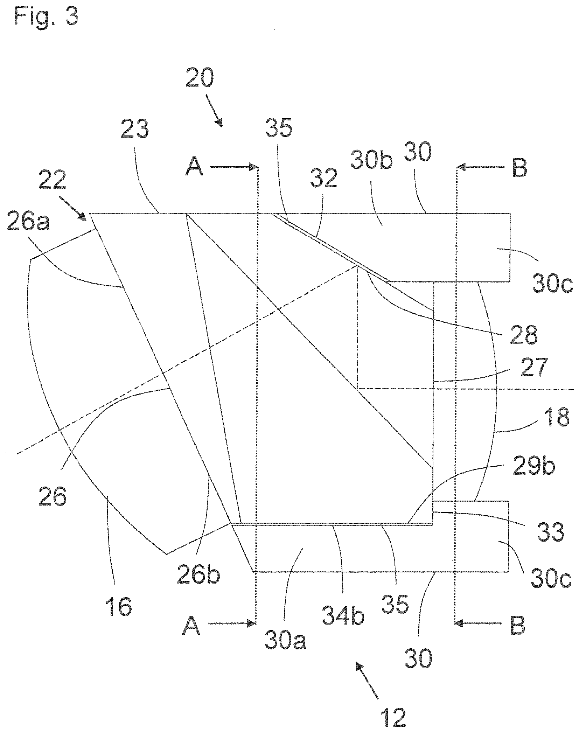

In Fig. 2 und Fig. 3 ist mit der gestrichelten Linie ein beispielhafter Strahlverlauf eines Lichtstrahls gezeigt, der im Wesentlichen aus dem Zentrum des Blickfelds des Endoskops 2 auf das Eintrittsfenster 16 trifft.

In Fig. 3 ist schematisch eine erfindungsgemäße Umlenkprismenbaugruppe 20 gezeigt. Im Vergleich zu dem zylinderförmigen Prismenhalter 30 gemäß dem Stand der Technik in Fig 2 umfasst der erfindungsgemäße Prismenhalter 30 in Fig. 3 einen ersten Teil 30a, einen zweiten Teil 30b und einen ringförmigen Anschlag 30c.

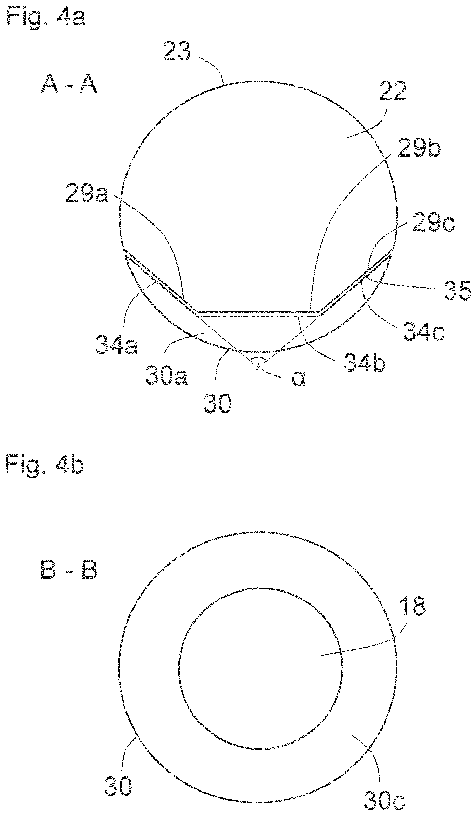

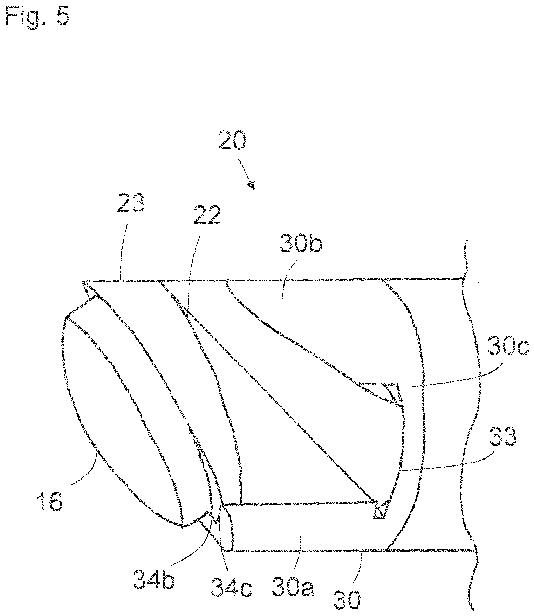

Der erste Teil 30a hat eine im Wesentlichen schlittenförmige Form und erstreckt sich von einer nahen Teilfläche 26b der Lichteintrittsfläche 26 zu einer Lichtaustrittsfläche 27 des Umlenkprismas 22. In Umfangsrichtung umschließt der erste Teil 30a den Außenumfang der Mantelfläche 23 in einem Winkel von etwa 150° (siehe Fig. 4a). Ein zweiter Teil 30b erstreckt sich von der Reflexionsfläche 28 in proximaler Richtung und hat eine im Wesentlichen keilförmige Form. Im Bereich der Austrittslinse 18 sind der erste Teil 30a und der zweite Teil 30b durch den ringförmigen Anschlag 30c verbunden (siehe Fig. 5).

Die Umlenkprismenbaugruppe 20 zeichnet sich dadurch aus, dass Bereiche des Prismenhalters 30 im Vergleich zu einem herkömmlichen Prismenhalter, wie er beispielhaft in Fig. 2 gezeigt ist, ausgespart sind. In den ausgesparten Bereichen ist das Umlenkprisma 22 entsprechend vergrößert. Dies betrifft unter anderem die Bereiche, die in der Darstellung von Fig. 3 oben liegen und an die entfernte Teilfläche 26a der Lichteintrittsfläche 26 angrenzen. Vor allem ist die Reflexionsfläche 28 größer. Durch die Vergrößerung des Umlenkprismas 22 können die optischen Eigenschaften des Endoskops 2 verbessert und dessen Lichtstärke vergrößert werden.

Gleichzeitig ist bei der Umlenkprismenbaugruppe 20 sichergestellt, dass trotz der Aussparung am Prismenhalter 30 das Umlenkprisma 22 präzise ausgerichtet und stabil in dem Prismenhalter 30 fixiert ist.

Zur Fixierung weist das Umlenkprisma 22 an der unteren Seite Bodenflächen 29a, 29b, 29c auf, von denen im Längsschnitt der Fig. 3 nur die Bodenfläche 29b sichtbar ist. Die Bodenflächen 29a, 29b, 29c stehen senkrecht auf der Lichtaustrittsfläche 27 und sind formkomplementär zu unteren Halteflächen 34a, 34b, 34c des ersten Teils 30a des Prismenhalters 30 ausgebildet. Die Bodenflächen 29a, 29b, 29c und die unteren Halteflächen 34a, 34b, 34c werden durch einen Klebespalt 35 getrennt. Der Klebespalt 35 ist notwendig, da die Bodenflächen 29a, 29b, 29c nur zur Fixierung, nicht aber zur Ausrichtung des Umlenkprismas 22 verwendet werden.

Der zweite Teil 30b des Prismenhalters 30 weist eine obere Haltefläche 32 auf. Diese ist formkomplementär zur Reflexionsfläche 28 und ist an dieser fixiert, beispielsweise mit einem geeigneten Klebstoff. Zwischen der oberen Haltefläche 32 und der Reflexionsfläche 28 befindet sich dazu ein Klebespalt 35.

Zur Ausrichtung des Umlenkprismas 22 liegt ein Bereich der Lichtaustrittsfläche 27 an einer Anlagefläche 33 des Anschlags 30c an. Auf diese Weise wird eine Verkippung des Umlenkprismas 32 minimiert oder gar ausgeschlossen.

Fig. 4a und 4b zeigen schematisch Querschnitte der Umlenkprismenbaugruppe 20 entlang der Linien A - A und B - B in Fig. 3. Wie Fig. 4a zeigt, umschließt der erste Teil 30a des Prismenhalters 30 die Mantelfläche 23 in einem Winkelbereich des Außenumfangs von etwa 150°.

Um eine genaue Zentrierung des Umlenkprismas 22 zu ermöglichen, ist die Mantelfläche 23 im nicht umschlossenen Teil des Außenumfangs kreisförmig. Bei dem in Fig. 4a gezeigten Beispiel sind dies etwa 210°.

An der Unterseite weist das Umlenkprisma 22 drei Bodenflächen 29a, 29b, 29c auf, die formkomplementär zu den unteren Halteflächen 34a, 34b, 34c des Prismenhalters 30 sind. Zwischen den Bodenflächen 29a, 29b, 29c und den Halteflächen 34a, 34b, 34c befindet sich der Klebespalt 35.

Um das Umlenkprisma 22 gegen ein Verrutschen unter der Einwirkung von Scherkräften zu schützen, ist es sinnvoll, die Bodenflächen 29a, 29b, 29c unter einem Summenwinkel α von etwa 90° anzuordnen. Die Summe der Winkel der aneinander angrenzenden unteren Halteflächen 34a, 34b, 34c beträgt also in etwa 90°. Diese Ausführung ist ein Kompromiss zwischen der Widerstandsfähigkeit gegenüber Scherkräften und der Möglichkeit zur Zentrierung des Umlenkprismas 22.

Ein Querschnitt entlang der Linie B - B, der in Fig. 4b gezeigt ist, verläuft durch einen Bereich der Umlenkprismenbaugruppe 20, in dem der Anschlag 30c des Prismenhalters 30 die Austrittslinse 18 umschließt. In dieser Querschnittsebene ist der Prismenhalter 30 kreisrund. Auf diese Weise wird sowohl die Stabilität des Prismenhalters 30 gewährleistet als auch eine radiale Ausrichtung, d.h. Zentrierung, ermöglicht. Dazu wird der kreisförmige Außenumfang des Prismenhalters 30 mit dem kreisförmigen Teil des Außenumfangs der Mantelfläche 23 des Umlenkprismas 22 zur Deckung gebracht und diese wiederum an der Endoskopachse ausgerichtet. Auf diese Weise ist eine präzise Ausrichtung des Umlenkprismas 22 möglich, die Vignettierung und Bildbeschnitt vermeidet.

Fig. 5 zeigt eine schematisch vereinfachte perspektivische Darstellung der Umlenkprismenbaugruppe 20, in der die Form des Prismenhalters 30 deutlich wird. Erkennbar ist die Form und Anordnung des schlittenförmigen ersten Teils 30a, des keilförmigen zweiten Teils 30b und des ringförmigen Anschlags 30c. Weiterhin ist zu erkennen, dass der Prismenhalter 30 die Mantelfläche 23 entlang keines Außenumfangs der Mantelfläche 23 vollständig umschließt, der erste Teil 30a also an keiner Stelle an den zweiten Teil 30b anschließt. Die Verbindung zwischen erstem Teil 30a und zweitem Teil 30b besteht allein in dem Anschlag 30c, der jedoch in proximaler Richtung am Umlenkprisma 22 anliegt und dieses nicht umschließt. Ebenfalls ist zu erkennen, dass das Umlenkprisma 22 mit der Lichtaustrittsfläche 27 an der Anlagefläche 33 des ringförmigen Anschlags 30c anliegt.

### Bezugszeichenliste

- 2: Endoskop
- 4: Handgriff
- 6: Schaft
- 8: distales Ende
- 10: Eintrittsfenster
- 12: distaler Endbereich
- 16: Eintrittslinse
- 18: Austrittslinse
- 20: Umlenkprismenbaugruppe
- 22: Umlenkprisma
- 22a - 22c: Teilprisma
- 23: Mantelfläche
- 26: Lichteintrittsfläche
- 26a: entfernte Teilfläche
- 26b: nahe Teilfläche
- 27: Lichtaustrittsfläche
- 28: Reflexionsfläche
- 29a - 29c: Bodenfläche
- 30: Prismenhalter
- 30a: erster Teil
- 30b: zweiter Teil
- 30c: Anschlag
- 32: obere Haltefläche
- 33: Anlagefläche
- 34a - 34c: untere Haltefläche
- 35: Klebespalt
- α: Winkel

## Patentansprüche

1. Umlenkprismenbaugruppe (20) für ein Endoskop (2) mit seitlicher Blickrichtung, umfassend einen Prismenhalter (30) und ein Umlenkprisma (22), welches in dem Prismenhalter (30) aufgenommen ist, wobei das Umlenkprisma (22) eine Lichtaustrittsfläche (27) und eine zu dieser schräg angeordnete, gegenüberliegende Lichteintrittsfläche (26) aufweist, zwischen denen sich eine Mantelfläche (23) erstreckt, wobei der Prismenhalter (30) das Umlenkprisma (22) dergestalt aufnimmt, dass der Prismenhalter (30) die Mantelfläche (23) des Umlenkprismas (22) bereichsweise umgibt, wobei der Prismenhalter (30) einen ersten Teil (30a) und einen zweiten Teil (30b) umfasst, wobei sich der erste Teil (30a) entlang einer gesamten Länge des Umlenkprismas (22) erstreckt und sich der zweite Teil (30b) entlang eines Abschnitts der Länge des Umlenkprismas (22) erstreckt, wobei die Länge eine Ausdehnung des Umlenkprismas (22) in Richtung einer Normalen auf der Lichtaustrittsebene (27) ist, wobei die Lichteintrittsfläche (26) durch die schräge Anordnung eine bezüglich der Lichtaustrittsfläche (27) nahe Teilfläche (26b) und eine entfernte Teilfläche (26a) aufweist, wobei sich der erste Teil (30a) des Prismenhalters (30) von der Lichtaustrittsfläche (27) zur nahen Teilfläche (26b) erstreckt und sich der zweite Teil (30b) des Prismenhalters (30) von der Lichtaustrittsfläche (27) in Richtung der entfernten Teilfläche (26a) erstreckt, **dadurch gekennzeichnet, dass** der zweite Teil (30b) des Prismenhalters (30) eine obere Haltefläche (32) umfasst, wobei eine Reflexionsfläche (28) des Umlenkprismas (22) an der oberen Haltefläche (32) fixiert ist.

2. Umlenkprismenbaugruppe (20) nach Anspruch 1, **dadurch gekennzeichnet, dass** der Prismenhalter (30) derart ausgestaltet ist, dass dieser das Umlenkprisma (22) an keinem der möglichen Außenumfänge des Umlenkprismas (22) vollständig umschließt, wobei die Außenumfänge auf der Mantelfläche (23) des Umlenkprismas (22) parallel zur Lichtaustrittsfläche (27) verlaufen.

3. Umlenkprismenbaugruppe (20) nach Anspruch 1, **dadurch gekennzeichnet, dass** der erste Teil (30a) des Prismenhalters (30) eine untere Haltefläche (34a, 34b, 34c) und das Umlenkprisma (22) eine formkomplementäre Bodenfläche (29a, 29b, 29c) umfasst, wobei die untere Haltefläche (34a, 34b, 34c) und die Bodenfläche (29a, 29b, 29c) senkrecht zur Lichtaustrittsfläche (27) angeordnet sind und die Bodenfläche (29a, 29b, 29c) an der unteren Haltefläche (34a, 34b, 34c) fixiert ist.

4. Umlenkprismenbaugruppe (20) nach Anspruch 3, **dadurch gekennzeichnet, dass** der erste Teil (30a) des Prismenhalters (30) wenigstens zwei aneinander angrenzende untere Halteflächen (34a, 34b, 34c) und das Umlenkprisma (22) den unteren Halteflächen (34a, 34b, 34c) zugeordnete Bodenflächen (29a, 29b, 29c) umfasst.

5. Umlenkprismenbaugruppe (20) nach Anspruch 4, **dadurch gekennzeichnet, dass** ein Summenwinkel (α), also die Summe der einzelnen Winkel der aneinander angrenzenden unteren Halteflächen (34a, 34b, 34c), zwischen 60° und 120°, insbesondere bei etwa 90° liegt.

6. Umlenkprismenbaugruppe (20) nach einem der Ansprüche 3 bis 5, **dadurch gekennzeichnet, dass** der erste Teil (30a) und der zweite Teil (30b) durch einen Anschlag (30c) des Prismenhalters (30) verbunden sind, wobei der Anschlag (30c) insbesondere ringförmig ist.

7. Umlenkprismenbaugruppe (20) nach Anspruch 6, **dadurch gekennzeichnet, dass** der Anschlag (30c) eine Anlagefläche (33) umfasst, die zur Lichtaustrittsfläche (27) des Umlenkprismas (22) planparallel ist, wobei die Lichtaustrittsfläche (27) bereichsweise an der Anlagefläche (33) anliegt.

8. Umlenkprismenbaugruppe (20) nach Anspruch 7, **dadurch gekennzeichnet, dass** zwischen unterer Haltefläche (34a, 34b, 34c) und zugeordneter Bodenfläche (29a, 29b, 29c) und/oder zwischen oberer Haltefläche (32) und zugeordneter Reflexionsfläche (28) ein Klebespalt (35) mit einer Spaltbreite ausgebildet ist, wobei die Spaltbreite so groß ist, dass unter Berücksichtigung von Fertigungstoleranzen die untere Haltefläche (34a, 34b, 34c) an keiner Stelle an der Bodenfläche (29a, 29b, 29c) und/oder die obere Haltefläche (32) an keiner Stelle an der Reflexionsfläche (28) anliegt, wenn die Lichtaustrittsfläche (27) bereichsweise an der Anlagefläche (33) anliegt.

9. Umlenkprismenbaugruppe (20) nach einem der Ansprüche 1 bis 8, **dadurch gekennzeichnet, dass** wenigstens ein Drittel, insbesondere wenigstens zwei Drittel, eines Außenumfangs der Mantelfläche (23) kreisförmig ist.

10. Umlenkprismenbaugruppe (20) nach einem der Ansprüche 1 bis 9, **dadurch gekennzeichnet, dass** wenigstens ein Drittel eines Außenumfangs des Prismenhalters (30), insbesondere ein vollständiger Außenumfang des Prismenhalters (30), kreisförmig ist.

11. Endoskop (2), umfassend eine Umlenkprismenbaugruppe (20) nach einem der Ansprüche 1 bis 10.

## Claims

1. A deflection prism assembly (20) for an endoscope (2) having a lateral viewing direction, comprising a prism holder (30) and a deflection prism (22) which is accommodated in the prism holder (30), wherein the deflection prism (22) has a light outlet surface (27) and an opposite light inlet surface (26) arranged obliquely thereto, and a lateral surface (23) extends between said inlet and outlet surfaces (26, 27), wherein the prism holder (30) accommodates the deflection prism (22) such that the prism holder (30) surrounds the lateral surface (23) of the deflection prism (22) in areas, wherein the prism holder (30) comprises a first part (30a) and a second part (30b), wherein the first part (30a) extends along an entire length of the deflection prism (22) and the second part (30b) extends along a section of the length of the deflection prism (22), wherein the length is an extension of the deflection prism (22) in direction of a normal on the light outlet plane (27), wherein due to the oblique arrangement, the light inlet surface (26) has a near partial surface (26b) and a distant partial surface (26a) with respect to the light outlet surface (27), wherein the first part (30a) of the prism holder (30) extends from the light outlet surface (27) to the near partial surface (26b) and the second part (30b) of the prism holder (30) extends from the light outlet surface (27) in the direction of the distant partial surface (26a) **characterized in that** the second part (30b) of the prism holder (30) comprises an upper holding surface (32), wherein a reflection surface (28) of the deflection prism (22) is fixed to the upper holding surface (32).

2. The deflection prism assembly (20) according to Claim 1, **characterized in that** the prism holder (30) is formed in such a way that the latter does not completely enclose the deflection prism (22) at any of the possible outer circumferences of the deflection prism (22), wherein the outer circumferences run parallel to the light outlet surface (27) on the lateral surface (23) of the deflection prism (22).

3. The deflection prism assembly (20) according to Claim 1, **characterized in that** the first part (30a) of the prism holder (30) comprises a lower holding surface (34a, 34b, 34c) and the deflection prism (22) comprises a bottom surface (29a, 29b, 29c) having a complementary shape, wherein the lower holding surface (34a, 34b, 34c) and the bottom surface (29a, 29b, 29c) are arranged perpendicularly to the light outlet surface (27) and the bottom surface (29a, 29b, 29c) is fixed to the lower holding surface (34a, 34b, 34c).

4. The deflection prism assembly (20) according to Claim 3, **characterized in that** the first part (30a) of the prism holder (30) comprises at least two adjacent lower holding surfaces (34a, 34b, 34c) and the deflection prism (22) comprises bottom surfaces (29a, 29b, 29c) associated with the lower holding surfaces (34a, 34b, 34c).

5. The deflection prism assembly (20) according to Claim 4, **characterized in that** a total angle (α), that is to say the total of the individual angles of the adjacent lower holding surfaces (34a, 34b, 34c), is between 60° and 120°, in particular is approximately 90°.

6. The deflection prism assembly (20) according to any one of Claims 3 to 5, **characterized in that** the first part (30a) and the second part (30b) are connected by a stop (30c) of the prism holder (30), wherein the stop (30c) is in particular annular.

7. The deflection prism assembly (20) according to Claim 6, **characterized in that** the stop (30c) comprises a contact surface (33) which is plane parallel to the light outlet surface (27) of the deflection prism (22), wherein the light outlet surface (27) rests on certain regions of the contact surface (33).

8. The deflection prism assembly (20) according to Claim 7, **characterized in that** an adhesive gap (35) having a gap width is configured between the lower holding surface (34a, 34b, 34c) and the associated bottom surface (29a, 29b, 29c) and/or between the upper holding surface (32) and the associated reflection surface (28), wherein the gap width is so large that, taking account of production tolerances, the lower holding surface (34a, 34b, 34c) does not rest on any point of the bottom surface (29a, 29b, 29c) and/or the upper holding surface (32) does not rest on any point of the reflection surface (28), if the light outlet surface (27) rests on certain regions of the contact surface (33).

9. The deflection prism assembly (20) according to any one of Claims 1 to 8, **characterized in that** at least one third, in particular at least two thirds, of an outer circumference of the lateral surface (23) is circular.

10. The deflection prism assembly (20) according to any one of Claims 1 to 9, **characterized in that** at least one third of an outer circumference of the prism holder (30), in particular a complete outer circumference of the prism holder (30), is circular.

11. An endoscope (2), comprising a deflection prism assembly (20) according to any one of Claims 1 to 10.

## Revendications

1. Ensemble formant prisme de déviation (20) pour un endoscope (2) ayant une direction de vision latérale, comprenant un support de prisme (30) et un prisme de déviation (22) qui est reçu dans le support de prisme (30), le prisme de déviation (22) présentant une surface (27) de sortie de lumière et une surface (26) d'entrée de lumière opposée et agencée de façon oblique par rapport à celle-ci, entre lesquelles s'étend une surface d'enveloppe (23), le support de prisme (30) recevant le prisme de déviation (22) de telle sorte que le support de prisme (30) entoure par endroits la surface d'enveloppe (23) du prisme de déviation (22), le support de prisme (30) comprenant une première partie (30a) et une deuxième partie (30b), la première partie (30a) s'étendant le long d'une longueur totale du prisme de déviation (22) et la deuxième partie (30b) s'étendant le long d'une partie de la longueur du prisme de déviation (22), la longueur étant une extension du prisme de déviation (22) dans la direction d'une perpendiculaire au plan (27) de sortie de la lumière, la surface (26) d'entrée de lumière présentant, du fait de l'agencement en oblique, une surface partielle (26b) proche de la surface (27) de sortie de lumière et une surface partielle (26a) éloignée, la première partie (30a) du support de prisme (30) s'étendant depuis la surface (27) de sortie de lumière vers la surface partielle proche (26b) et la deuxième partie (30b) du support de prisme (30) s'étendant depuis la surface (27) de sortie de lumière vers la surface partielle éloignée (26a), **caractérisé en ce que** la deuxième partie (30b) du support de prisme (30) comprend une surface de maintien supérieure (32), une surface réfléchissante (28) du prisme de déviation (22) étant fixe par rapport à la surface de maintien supérieure (32).

2. Ensemble formant prisme de déviation (20) selon la revendication 1, **caractérisé en ce que** le support de prisme (30) est conçu de telle sorte qu'il n'entoure complètement le prisme de déviation (22) sur aucune des circonférences extérieures possibles du prisme de déviation (22), les circonférences extérieures s'étendant sur la surface d'enveloppe (23) du prisme de déviation (22) parallèlement à la surface (27) de sortie de lumière.

3. Ensemble formant prisme de déviation (20) selon la revendication 1, **caractérisé en ce que** la première partie (30a) du support de prisme (30) comprend une surface de maintien inférieure (34a, 34b, 34c) et le prisme de déviation (22) comprend une surface de partie inférieure (29a, 29b, 29c) de forme complémentaire, la surface de maintien inférieure (34a, 34b, 34c) et la surface de partie inférieure (29a, 29b, 29c) étant agencées perpendiculairement à la surface (27) de sortie de lumière, et la surface de partie inférieure (29a, 29b, 29c) étant fixe par rapport à la surface de maintien inférieure (34a, 34b, 34c).

4. Ensemble formant prisme de déviation (20) selon la revendication 3, **caractérisé en ce que** la première partie (30a) du support de prisme (30) comprend au moins deux surfaces de maintien inférieures (34a, 34b, 34c) adjacentes l'une à l'autre et le prisme de déviation (22) comprend des surfaces de partie inférieure (29a, 29b, 29c) associées aux surfaces de maintien inférieures (34a, 34b, 34c).

5. Ensemble formant prisme de déviation (20) selon la revendication 4, **caractérisé en ce qu'**un angle de somme (α), c'est-à-dire la somme des angles individuels des surfaces de maintien inférieures (34a, 34b, 34c) adjacentes les unes aux autres, est compris entre 60° et 120°, en particulier est d'environ 90°.

6. Ensemble formant prisme de déviation (20) selon l'une des revendications 3 à 5, **caractérisé en ce que** la première partie (30a) et la deuxième partie (30b) sont reliées par une butée (30c) du support de prisme (30), la butée (30c) étant notamment annulaire.

7. Ensemble formant prisme de déviation (20) selon la revendication 6, **caractérisé en ce que** la butée (30c) comprend une surface d'appui (33) qui est plane et parallèle à la surface (27) de sortie de lumière du prisme de déviation (22), la surface (27) de sortie de lumière étant en appui par endroits sur la surface d'appui (33).

8. Ensemble formant prisme de déviation (20) selon la revendication 7, **caractérisé en ce qu'**entre la surface de maintien inférieure (34a, 34b, 34c) et la surface de partie inférieure (29a, 29b, 29c) associée et/ou entre la surface de maintien supérieure (32) et la surface de réflexion (28) associée est formée un interstice (35) pour adhésif ayant une largeur d'interstice, la largeur d'interstice étant grande au point que, compte tenu des tolérances de fabrication, la surface de maintien inférieure (34a, 34b, 34c) ne s'appuie en aucun endroit sur la surface de partie inférieure (29a, 29b, 29c) et/ou que la surface de maintien supérieure (32) ne s'appuie en aucun endroit sur la surface de réflexion (28) lorsque la surface (27) de sortie de lumière s'appuie par endroits sur la surface d'appui (33).

9. Ensemble formant prisme de déviation (20) selon l'une des revendications 1 à 8, **caractérisé en ce qu'**au moins un tiers, en particulier au moins deux tiers, d'une circonférence extérieure de la surface d'enveloppe (23) est circulaire.

10. Ensemble formant prisme de déviation (20) selon l'une des revendications 1 à 9, **caractérisé en ce qu'**au moins un tiers d'une périphérie extérieure du support de prisme (30), en particulier une périphérie extérieure complète du support de prisme (30), est circulaire.

11. Endoscope (2) comprenant un ensemble formant prisme de déviation (20) selon l'une quelconque des revendications 1 à 10.
